# EUROPÄISCHE PATENTANMELDUNG

(11) **EP 4 825 347 A1**
(43) Veröffentlichungstag der Anmeldung: **21.10.2026**
(21) Anmeldenummer: 25171074.5
(22) Anmeldetag: 16.04.2025
(51) Int. Cl.: A61F 2/52

(54) **BRUSTPROTHESE MIT VERSTÄRKTER RÜCKSEITE**

(71) Anmelder: Edelweiss Basics GmbH & Co. KG, 83098 Brannenburg (DE)
(72) Erfinder: WEBER-UNGER, Georg, 6330 Kufstein (AT)
(74) Vertreter: Kador & Partner Part mbB

(57) **Zusammenfassung**

Gegenstand der Erfindung ist eine Brustprothese (1) umfassend einen schalenförmigen Körper (2), der eine konvexe Vorderseite (3) sowie eine an die Vorderseite anschließende konkave Rückseite (4) aufweist, wobei an der konkaven Rückseite (4) eine oder mehrere stützende Verstärkungen angeordnet sind, die als ein oder mehrere umlaufende Bänder (4b, 2b) ausgeführt sind, die in einer Rückansicht des schalenförmigen Körpers (2) im Wesentlichen parallel zu einer Außenkontur des schalenförmigen Körpers (2) verlaufen, und/oder als Rippen (11) ausgeführt sind, die sich in einer Rückansicht des schalenförmigen Körpers (2) in vertikaler Richtung parallel zueinander oder sternförmig von einem Innenraum (10) des schalenförmigen Körpers (2) in Richtung von dessen Außenrändern (7a, 7b, 8, 9) erstrecken.

## Beschreibung

Die Erfindung betrifft eine Brustprothese, insbesondere eine Brustprothese zur Erstversorgung, zum Tragen in einer linken oder rechten Büstenschale eines Büstenhalters oder Korseletts nach einer Brustamputation bei Frauen, insbesondere eine Brustprothese mit einer verstärkten Rückseite.

Direkt nach der Brustoperation ist das Operationsgebiet und das umliegende Gewebe der Patientin sehr empfindlich. In der Regel erhalten Brustkrebspatientinnen ihre Erstversorgung noch im Krankenhaus. Die Brustprothese zur Erstversorgung wird meist für wenige Wochen getragen, bis die Wunde vollständig verheilt ist. Probleme ergeben sich dann, wenn die Brustprothese während des Tragens am operierten Gewebe drückt und/oder reibt. Dies kann zu Schmerzen führen und die Heilung nach der Brust-OP beeinträchtigen. Insofern werden an Brustprothesen zur Erstversorgung hohe Anforderungen gestellt. Sie sollen nicht nur stabil sein, sondern zugleich auch einen hohe Tragekomfort aufweisen.

Brustprothesen mit verstärkter Rückseite sind beispielsweise aus EP 0 657 148 A1 bekannt. Diese offenbart eine Brustprothese zum Tragen im Oberteil eines Badebekleidungsstücks besteht aus einem weich-elastischen, schalenförmigen Kunststoffkörper, dessen dem Körper der Trägerin zugewandte Rückseite eine Aushöhlung und mehrere aus der Oberfläche der Aushöhlung vorspringende Versteifungsrippen aufweist, die sich geradlinig und ununterbrochen zwischen dem oberen und unteren Rand der Aushöhlung erstrecken. Die Versteifungsrippen weisen bevorzugt eine Rückseite auf, die in einem mittleren Längsabschnitt konkav gebogen ist, um eine Anlage der Rippen an einer Operationsnarbe zu verhindern.

Mittels der in EP 0 657 148 A1 gezeigten Versteifungsrippen kann jedoch nicht vermieden werden, dass die Brustprothese in einem Büstenhalter oder Korsett unerwünscht verformt, insbesondere plattgedrückt wird.

Der Erfindung liegt die Aufgabe zugrunde, eine Brustprothese bereitzustellen, welche sowohl Stabilität als auch Tragekomfort der Prothese erhöht.

Zur Lösung der Aufgabe werden die Merkmale des unabhängigen Anspruchs 1 vorgeschlagen. Vorteilhafte Ausgestaltungen finden sich in den abhängigen Ansprüchen.

Gegenstand der Erfindung ist eine Brustprothese umfassend einen schalenförmigen Körper aus einem elastischen Thermoplast, der eine konvexe Vorderseite sowie eine an die Vorderseite anschließende konkave Rückseite aufweist.

Bei der erfindungsgemäßen Brustprothese kann es sich insbesondere um eine symmetrische Brustprothese handelt, deren Symmetrieachse mittig und vertikal durch die Brustprothese verläuft.

Die konvexe Vorderseite des schalenförmigen Körpers bildet insbesondere eine Form einer weiblichen Brust nach, während die konkave Rückseite, die einem Körper einer Trägerin zugewandt ist, eine Aushöhlung aufweist und dazu eingerichtet ist, nur auf ihren Rändern am Körper der Trägerin aufzuliegen, wodurch die Brustprothese eine gute Belüftung von darunter liegender Haut und Platz für verbliebenes Brustgewebe bietet.

An der konkaven Rückseite des schalenförmigen Körpers sind eine oder mehrere stützende Verstärkungen angeordnet, die als ein oder mehrere umlaufende Bänder und/oder als Rippen ausgeführt sind. Die stützenden Verstärkungen können insbesondere in die konkave Rückseite integriert sein.

Die erfindungsgemäße Brustprothese mit schalenförmigem Körper weist den Vorteil auf, dass durch die stützenden Verstärkungen, deren Stabilität insbesondere gegenüber einer Druckkraft auf der Vorderseite erhöht und somit einem "Plattdrücken" der Prothese entgegengewirkt wird. Die stützenden Verstärkungen, insbesondere die ein oder mehrere umlaufende Bänder, verhindern ein Auseinanderspreizen der Prothese. Da die Verstärkungen nur wenig Material benötigen, tritt nahezu keine Gewichtserhöhung auf. Zudem erlaubt die konkave Form der Rückseite eine ausreichende Belüftung von unter der Prothese befindlicher Haut der Trägerin, wodurch der Tragekomfort gesteigert werden kann. Mit anderen Worten stellt die Erfindung eine Brustprothese mit erhöhter Stabilität bereit und verbesserter Belüftung bereit, die keine nennenswerte Gewichtszunahme zur Folge hat und eine unerwünschte Verformung der Prothese z.B. in einem Büstenhalter zuverlässig vermeiden kann.

Die Rippen erstrecken sich in einer Rückansicht des schalenförmigen Körpers in vertikaler Richtung parallel zueinander oder sternförmig von einem Innenraum des schalenförmigen Körpers in Richtung von dessen Außenrändern. Insbesondere können sich die sternförmigen Rippen von einem tiefsten Punkt des schalenförmigen Körpers auf der konkaven Rückseite nach außen erstrecken.

Gemäß einer Ausführungsform kann der schalenförmige Körper einstückig ausgebildet sein. Dabei kann dieser gegossen oder aus einem Materialblock geschnitten sein. Insbesondere kann der schalenförmige Körper aus einem elastischen Thermoplast, Kautschuk, Naturkautschuk oder Silikonkautschuk gefertigt sein.

In einer Ausführungsform können die Rippen zudem zusätzliches Material zur Beschwerung, beispielsweise ein Granulat, umfassen, wodurch deren Stabilität nochmals erhöht werden kann.

In einer weiteren Ausführungsform kann auf einer Seite der Rippen, die dem Körper der Trägerin zugewandt ist, eine Vielzahl von Noppen angeordnet sein. Dadurch kann die Belüftung der Haut nochmals verbessert und zudem eine Lymphdrainage im Unterhautgewebe stimuliert werden.

Das eine oder die mehreren umlaufenden Bänder verlaufen in einer Rückansicht des schalenförmigen Körpers im Wesentlichen parallel zu einer Außenkontur desselben. Insbesondere bildet jedes Band einen Hinterschnitt mit der konkaven Rückseite des schalenförmigen Körpers aus und kann somit auch als hinterschnittener (innerer) Rand der Rückseite des schalenförmigen Körpers bezeichnet werden. Das eine oder die mehren Bänder können z.B. durch Verschweißen eines entsprechend geformten elastischen thermoplastischen Materials auf einem Umfang der konkaven Rückseite gebildet sein. Es ist jedoch ebenso möglich, dass der schalenförmige Körper mit dem ein oder den mehreren Bändern einstückig ausgeführt ist.

Gemäß einer Ausführungsform kann sich das eine oder die mehreren umlaufenden Bänder von der konkaven Rückseite des schalenförmigen Körpers in dessen Innenraum erstrecken. Mit anderen Worten kann das umlaufende Band bzw. können die umlaufenden Bänder eine vorbestimmte Breite aufweisen, die in den Innenraum des schalenförmigen Körpers hineinragt.

In einer weiteren Ausführungsform kann sich ein erstes umlaufendes Band an die Außenränder des schalenförmigen Körpers anschließen, und ein zweites umlaufendes Band kann an einem inneren Umfang der konkaven Rückseite des schalenförmigen Körpers angeordnet ist. Mit anderen Worten kann das erste umlaufende Band angrenzend an eine Auflagefläche der Brustprothese angebracht sein, um dort eine erhöhte Stabilität bereitzustellen. Das zweite umlaufende Band kann hingegen beabstandet von der Auflagefläche weiter im Innenraum des schalenförmigen Körpers angebracht sein.

Gemäß einer Ausführungsform kann zwischen dem ersten und dem zweiten umlaufenden Band ein erster Hohlraum in dem Innenraum des schalenförmigen Körpers ausgebildet sein. Über den Hohlraum bilden das erste und zweite Band eine Art Sicke auf der konkaven Rückseite des schalenförmigen Körpers aus, welche dort eine Versteifung bewirken und einer Verformung der Brustprothese gegenüber einer Druckkraft auf der Vorderseite entgegenwirken kann.

In einer weiteren Ausführungsform kann angrenzend an das zweite umlaufende Band ein zweiter Hohlraum in dem schalenförmigen Körper ausgebildet sein, der dazu eingerichtet ist, eine Füllmasse aufzunehmen. Mit anderen Worten kann die Füllmasse in einem vorderen Bereich der Brustprothese angeordnet sein, welcher nach hinten (in Richtung des Körpers der Trägerin) mit dem zweiten Band abschließt bzw. von dem zweiten Band begrenzt wird. Auf diese Weise kann die Füllmasse von dem zweiten umlaufenden Band stabilisiert werden. Dazu kann das zweite umlaufende Band eine Kontur aufweisen, die sich an eine Kontur der Füllmasse anlegt.

Die Füllmasse kann eine weich-elastische Silikonkautschukmasse umfassen, welche von einer Folie, z.B. aus Polyurethan. ummantelt sein kann. Es ist ebenso möglich, dass die Füllmasse ohne eine Folienummantelung in dem schalenförmigen Körper, insbesondere in dem zweiten Hohlraum angebracht ist.

Gemäß einer Ausführungsform können in der Füllmasse Versteifungsrippen angeordnet sein, die sich in vertikaler Richtung erstrecken. Die Versteifungsrippen in der Füllmasse können insbesondere eine zusätzliche Stabilisierung im vorderen Bereich der Brustprothese bewirken.

In einer weiteren Ausführungsform kann der schalenförmige Körper in einer Vorderansicht eine dreiecksähnliche Form haben und einen oberen Ausläufer, einen linken Ausläufer und einen rechten Ausläufer aufweisen. Betrachtet man die Vorderseite der Brustprothese, so ist der links angeordnete seitliche Ausläufer der linke Ausläufer, und entsprechend der rechts angeordnete der rechte Ausläufer. Mit oberem Ausläufer ist jener Ausläufer gemeint, der im Tragezustand der Brustprothese zur Schulter der Trägerin weist. Der linke Seitenrand kann sich von dem oberen Ausläufer bis zu dem linken Ausläufer erstrecken, während sich der rechte Seitenrand von dem oberen Ausläufer bis zu dem rechten Ausläufer erstrecken kann. Aufgrund der Symmetrie der Brustprothese können die beiden seitlichen Ausläufer einander spiegelbildlich in Form und Größe entsprechen.

Dabei können die Außenränder des schalenförmigen Körpers einen oberen Außenrand an dem oberen Ausläufer, eine linken Außenrand an dem linken Ausläufer, einen rechten Außenrand an dem rechten Ausläufer und einen Unterbrustrand umfassen, der sich zwischen dem linken Ausläufer und dem rechten Ausläufer erstreckt.

Der linke und der rechte Außenrand der Brustprothese können jeweils einen konkaven Verlauf aufweisen. Mit anderen Worten können sowohl der linke als auch der rechte Außenrand einen nach innen gekrümmten Verlauf aufweisen.

Der Unterbrustrand kann insbesondere einen unteren Außenrand des schalenförmigen Körpers bilden und zumindest ein Teil eines Verlaufs des Unterbrustrands kann einem Kreisbogen entsprechen. Ebenso kann ein Teil eines Verlaufs des oberen Außenrands einem Kreisbogen entsprechen.

Weitere Einzelheiten, Merkmale und Vorteile des Gegenstands der Erfindung ergeben sich aus der nachfolgenden Beschreibung der zugehörigen Figuren, in welchen beispielhaft eine bevorzugte Ausführungsform der Erfindung dargestellt ist.

Darin zeigen
Figur 1a eine Vorderansicht eines ersten Ausführungsbeispiels der erfindungsgemäßen Brustprothese,
Figur 1b eine Seitenansicht der in Figur 1a abgebildeten Brustprothese,
Figur 1c einen Längsschnitt der in Figur 1a abgebildeten Brustprothese entlang der in Figur 1a dargestellten Symmetrieachse,
Figur 2a eine Rückansicht eines zweiten Ausführungsbeispiels der erfindungsgemäßen Brustprothese, und
Figur 2b eine Seitenansicht der in Figur 2a abgebildeten Brustprothese.

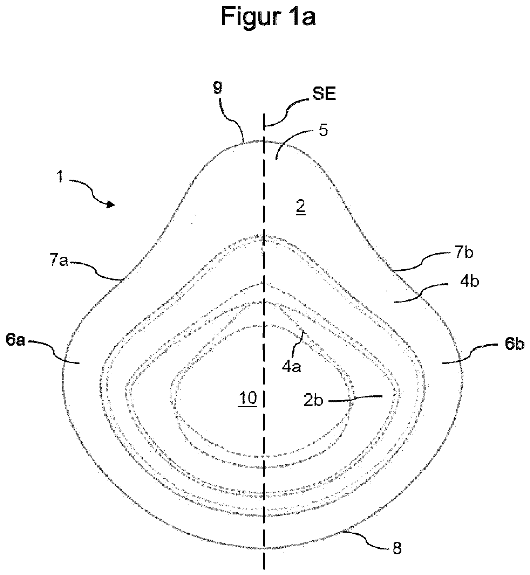

Figur 1a zeigt eine Vorderansicht eines Ausführungsbeispiels der erfindungsgemäßen Brustprothese 1, welche einen schalenförmigen Körper 2 umfasst, der eine konvexe Vorderseite 3 und eine konkave Rückseite 4 aufweist. Der schalenförmige Körper 2 kann in diesem Ausführungsbeispiel insbesondere aus einem elastischen Thermoplast bestehen. Es ist jedoch ebenso möglich, dass der schalenförmige Körper 2 aus Kautschuk, Naturkautschuk oder Silikonkautschuk gefertigt ist.

Die vorliegend gezeigte Brustprothese 1 bzw. deren schalenförmiger Körper 2 weist in der in Figur 1a gezeigten Vorderansicht eine dreiecksähnliche Form mit einem oberen Ausläufer 5 und zwei seitlichen Ausläufern 6a, 6b auf. Dabei umfasst der obere Ausläufer 5 einen oberen Außenrand 9 mit einem zumindest teilweise bogenförmigen Verlauf. Der linke Ausläufer 6a und der rechte Ausläufer 6b umfassen jeweils einen Außenrand 7a, 7b mit einem konkaven Verlauf, welche den oberen Außenrand 9 mit einem Unterbrustrand 8 verbinden, der einen unteren Außenrand des schalenförmigen Körpers 2 bildet. Der Unterbrustrand 8 weist vorliegend ebenfalls einen zumindest teilweise bogenförmigen Verlauf auf und erstreckt sich zwischen dem linken Ausläufer 6a und dem rechten Ausläufer 6b.

Eine Symmetrieebene SE verläuft mittig und vertikal durch den schalenförmigen Körper 2 der Brustprothese 1.

Des Weiteren sind in Figur 1a ein erstes umlaufendes Band 4a sowie ein zweites umlaufendes Band 2b angedeutet, die als stützende Verstärkungen auf der konkaven Rückseite 4 des schalenförmigen Körpers 2 angebracht sind.

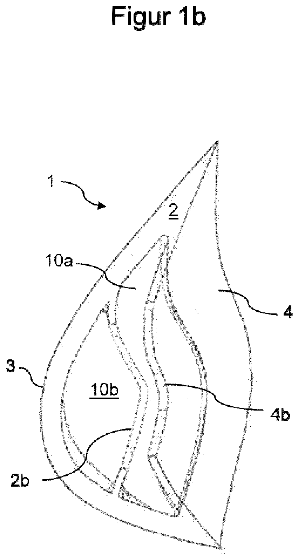

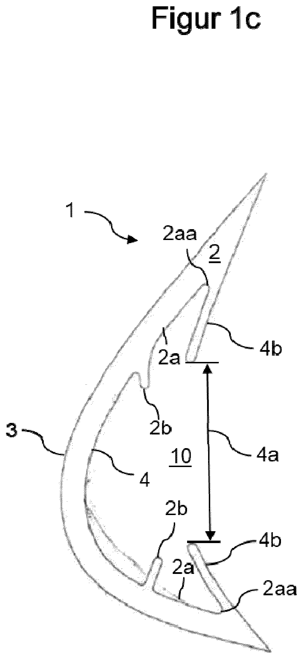

Das erste und zweite umlaufende Band 4b, 2b werden in Verbindung mit den Figuren 1b und 1c näher erläutert

Figur 1b zeigt schematisch eine Seitenansicht und Figur 1c zeigt schematisch einen Längsschnitt entlang der Symmetrieachse SE der in Figur 1a abgebildeten Brustprothese 1. In den Figuren 1b und 1c ist insbesondere die konvexe Vorderseite 3 sowie die konkave Rückseite 4 des schalenförmigen Körpers 2 der Brustprothese 1 gut erkennbar. Das erste umlaufende Band 4b ist angrenzend an die Außenränder 7a, 7b, 8, 9 des schalenförmigen Körpers 2 an der konkaven Rückseite 4 angebracht und kann mit dieser z.B. durch Verschweißen des elastischen thermoplastischen Materials verbunden sein. Das erste umlaufende Band 4b bildet eine Öffnung 4a aus, deren Mittelpunkt auf der Symmetrieachse des schalenförmigen Körpers 2 liegt und deren Form im Wesentlichen parallel zu dessen Außenkontur ist (siehe auch Figur 1a). Insbesondere bildet das erste umlaufende Band 4b mit einem Bereich 2a der konkave Rückseite 4 einen Hinterschnitt aus, der sich von der Öffnung 4a bis zu einem äußeren Ende 2aa des Bereichs 2a erstreckt.

Das zweite umlaufende Band 2b ist an einem weiter innen liegenden Umfang der konkaven Rückseite 4 des schalenförmigen Körpers 2 im Innenraum 10 des schalenförmigen Körpers 2 angeordnet. Insbesondere bildet das zweite umlaufende Band 2b einen Hinterschnitt mit einem Bereich 2c der konkaven Rückseite 4 des schalenförmigen Körpers 2 aus. Analog zu dem ersten Band 4b kann auch das zweite Band 2b z.B. durch Verschweißen eines entsprechend geformten elastischen thermoplastischen Materials auf einem Umfang der der konkaven Rückseite 4 gebildet sein. Es ist jedoch ebenso möglich, dass das erste und zweite Band 4b, 2b und der schalenförmige Körper 2 einstückig ausgebildet sind.

Zwischen dem ersten und zweiten Band 4a, 2a ist ein erster Hohlraum 10a in dem Innenraum 10 angeordnet, der eine Art Sicke auf der konkaven Rückseite 4 des schalenförmigen Körpers 4 ausbildet. Diese führt zu einer Versteifung des schalenförmigen Körpers 2, wodurch eine unerwünschte Verformung, insbesondere eine Abflachung der elastischen Brustprothese bei Druckeinwirkung, z.B. in einem Büstenhalter vermieden werden kann. Dabei ist das erste umlaufende Band 4b in der in Figur 1c gezeigten Schnittdarstellung nach vorne (in Richtung des Innenraums 10) geneigt, wodurch der schalenförmige Körper 2 lediglich auf seinen Außenrändern 7a, 7b, 8, 9 an einem Körper einer Trägerin aufliegt. Dies ermöglicht eine gute Belüftung von darunter liegender Haut und bietet Platz für verbliebenes Brustgewebe.

Das zweite umlaufende Band 2b ist in der in Figur 1c gezeigten Schnittdarstellung nach hinten (in Richtung der Öffnung 4a) geneigt und begrenzt einen zweiten Hohlraum 10b, in dem eine Füllmasse (nicht dargestellt) angeordnet sein kann. Dabei kann das zweite umlaufende Band 2b eine Kontur aufweisen, die sich an eine Kontur der Füllmasse anlegt, so dass diese in dem zweiten Hohlraum 10b stabilisiert werden kann. Die Füllmasse kann eine weich-elastische Silikonkautschukmasse umfassen, und in der Füllmasse können Versteifungsrippen angeordnet sein, die sich in vertikaler Richtung erstrecken (nicht dargestellt). Die Füllmasse kann mit einer Folie, z.B. aus Polyurethan, ummantelt sein. Es ist jedoch ebenso möglich, dass die Füllmasse ohne eine Folienummantelung in dem schalenförmigen Körper 2, insbesondere in dem zweiten Hohlraum 10b angebracht ist.

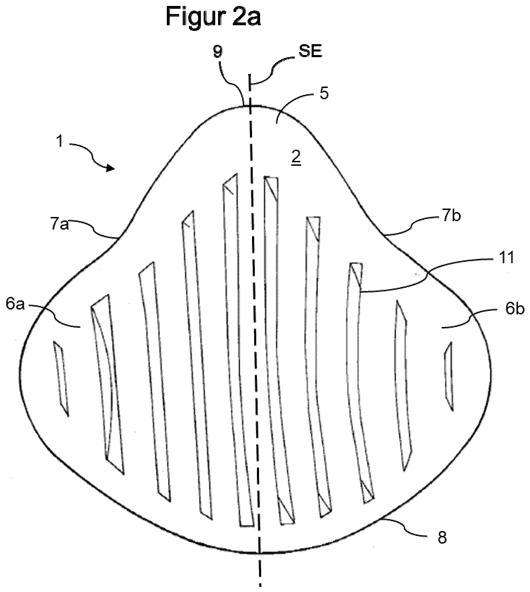

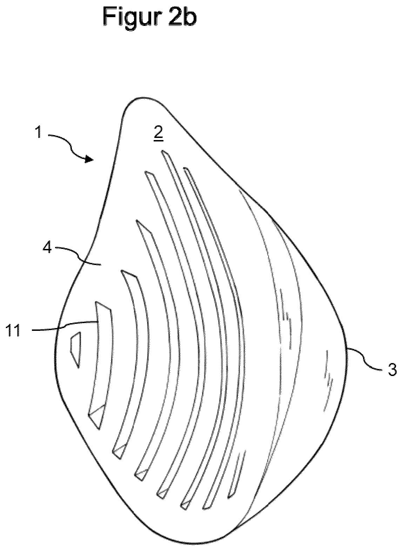

Figur 2a zeigt eine Rückansicht eines zweiten Ausführungsbeispiels der erfindungsgemäßen Brustprothese 1 und Figur 2b zeigt eine Seitenansicht der in Figur 2a abgebildeten Brustprothese 1.

Analog zu dem in den Figuren 1a bis 1c gezeigten Ausführungsbeispiel umfasst die vorliegende Brustprothese 1 einen schalenförmigen Körper 2, der eine konvexe Vorderseite 3 und eine konkave Rückseite 4 aufweist. Der schalenförmige Körper 2 kann in dieser Ausführungsform insbesondere aus Naturkautschuk bestehen. Es ist jedoch ebenso möglich, dass der schalenförmige Körper aus Silikonkautschuk oder einem elastischen Thermoplast gefertigt ist.

Das vorliegend gezeigte Ausführungsbeispiel der Brustprothese 1 bzw. deren schalenförmiger Körper 2 weist in der in Figur 1a gezeigten Rückansicht ebenfalls eine dreiecksähnliche Form mit einem oberen Ausläufer 5 und zwei seitlichen Ausläufern 6a, 6b auf, deren Außenränder 7a, 7b, 8, 9 entsprechend dem ersten Ausführungsbeispiel geformt sind.

Im Unterschied zu dem ersten Ausführungsbeispiel sind die stützenden Verstärkungen vorliegend als Rippen 11 ausgeführt, die sich in der gezeigten Rückansicht des schalenförmigen Körpers 2 auf dessen konkaver Rückseite 2 in vertikaler Richtung parallel zueinander erstrecken. Der schalenförmigen Körper 2 ist vorliegend einstückig ausgeführt und kann insbesondere gegossen oder aus einem geschäumten Naturkautschukblock geschnitten sein. Zur weiteren Verstärkung können die Rippen Material zur Beschwerung, z.B. ein Granulat, enthalten. Auf einer Seite der Rippen, die dem Körper der Trägerin zugewandt ist, kann zusätzlich eine Vielzahl von Noppen (nicht dargestellt) angeordnet sein. Dadurch kann die Belüftung der Haut nochmals verbessert und zudem eine Lymphdrainage im Unterhautgewebe stimuliert werden.

Anstatt der gezeigten vertikal verlaufenden Rippen können diese sich auch sternförmig von einem Punkt auf der konkaven Rückseite 4 des schalenförmigen Körpers 2 zu dessen Außenrändern 7a, 7b, 8, 9 erstrecken. Beispielsweise können sich die sternförmigen Rippen ausgehend von einem tiefsten Punkt des schalenförmigen Körpers 2 auf der konkaven Rückseite 4 nach außen erstrecken. Auf diese Weise können die stützenden Verstärkungen eine gesamte Fläche der konkaven Rückseite 4 erfassen.

### Bezugszeichenliste

- 1: Brustprothese
- 2: Schalenförmiger Körper der Brustprothese (1)
- 2a: Bereich der konkaven Rückseite (4) des schalenförmigen Körpers (2)
- 2b: Zweites umlaufendes Band
- 2c: Bereich der konkaven Rückseite (4) des schalenförmigen Körpers (2)
- 3: Konvexe Vorderseite des schalenförmigen Körpers (2)
- 4: Konkave Rückseite des schalenförmigen Körpers (2)
- 4a: Öffnung
- 4b: Erstes umlaufendes Band
- 5: Oberer Ausläufer
- 6a: Linker Ausläufer
- 6b: Rechter Ausläufer
- 7a: Linker Außenrand
- 7b: Rechter Außenrand
- 9: Oberer Außenrand
- 8: Unterbrustrand
- 11: Rippen
- SE: Symmetrieachse

## Patentansprüche

1. Brustprothese (1) umfassend einen schalenförmigen Körper (2) der eine konvexe Vorderseite (3) sowie eine an die Vorderseite anschließende konkave Rückseite (4) aufweist, wobei an der konkaven Rückseite (4) eine oder mehrere stützende Verstärkungen angeordnet sind, die
als ein oder mehrere umlaufende Bänder (4b, 2b) ausgeführt sind, die in einer Rückansicht des schalenförmigen Körpers (2) im Wesentlichen parallel zu einer Außenkontur des schalenförmigen Körpers (2) verlaufen, und/oder
als Rippen (11) ausgeführt sind, die sich in einer Rückansicht des schalenförmigen Körpers (2) in vertikaler Richtung parallel zueinander oder sternförmig von einem Innenraum (10) des schalenförmigen Körpers (2) in Richtung von dessen Außenrändern (7a, 7b, 8, 9) erstrecken.

2. Brustprothese (1) nach Anspruch 1, wobei der schalenförmige Körper (2) einstückig ausgebildet, insbesondere gegossen oder aus einem Materialblock geschnitten, ist.

3. Brustprothese (1) nach Anspruch 1 oder 2, wobei die Rippen (11) Material zur Beschwerung umfassen.

4. Brustprothese (1) nach einem der Ansprüche 1 bis 3, wobei auf einer Seite der Rippen (11), die einem Körper einer Trägerin zugewandt ist, eine Vielzahl von Noppen angeordnet ist.

5. Brustprothese (1) nach Anspruch 1 oder 2, wobei sich das eine oder die mehreren umlaufenden Bänder (4b, 2b) von der konkaven Rückseite (4) des schalenförmigen Körpers (2) in dessen Innenraum (10) erstrecken.

6. Brustprothese (1) nach Anspruch 5, wobei ein erstes umlaufendes Band (4b) an die Außenränder (7a, 7b, 8, 9) des schalenförmigen Körpers (2) anschließt, und ein zweites umlaufendes Band (2b) an einem inneren Umfang der konkaven Rückseite (4) des schalenförmigen Körpers (2) angeordnet ist.

7. Brustprothese (1) nach Anspruch 6, wobei zwischen dem ersten und dem zweiten umlaufenden Band (4b, 2b) ein erster Hohlraum (10a) in dem Innenraum (10) des schalenförmigen Körpers (2) ausgebildet ist.

8. Brustprothese (1) nach Anspruch 6 oder 7, wobei angrenzend an das zweite umlaufende Band (2b) ein zweiter Hohlraum (10b) in dem schalenförmigen Körper (2) ausgebildet ist, der dazu eingerichtet ist, eine Füllmasse aufzunehmen.

9. Brustprothese (1) nach Anspruch 8, wobei die Füllmasse eine weich-elastische Silikonkautschukmasse umfasst.

10. Brustprothese (1) nach Anspruch 8 oder 9, wobei in der Füllmasse Versteifungsrippen angeordnet sind, die sich in vertikaler Richtung erstrecken.

11. Brustprothese (1) nach mindestens einem der vorstehenden Ansprüche, wobei der schalenförmige Körper (2) in einer Vorderansicht eine dreiecksähnliche Form hat und einen oberen Ausläufer (5), einen linken Ausläufer (6a) und einen rechten Ausläufer (6b) aufweist.

12. Brustprothese (1) nach Anspruch 11, wobei die Außenränder (7a, 7b, 8, 9) des schalenförmigen Körpers einen oberen Außenrand (9) an dem oberen Ausläufer (5), eine linken Außenrand (7a) an dem linken Ausläufer (6a), einen rechten Außenrand (7b) an dem rechten Ausläufer (6b) und einen Unterbrustrand (8) umfassen.

13. Brustprothese (1) nach Anspruch 12 zumindest ein Teil eines Verlaufs des oberen Außenrands (9) und ein zumindest ein Teil eines Verlaufs des Unterbrustrands (8) einem Kreisbogen entspricht.

14. Brustprothese (1) nach einem der vorstehenden Ansprüche, wobei der schalenförmige Körper (2) aus einem elastischen Thermoplast, Kautschuk, Naturkautschuk oder Silikonkautschuk gefertigt ist.

15. Verwendung von elastischem Thermoplast, Kautschuk, Naturkautschuk oder Silikonkautschuk als Material für eine Brustprothese.
